# EUROPÄISCHE PATENTSCHRIFT

(11) **EP 4 583 817 B1**
(45) Veröffentlichungstag und Bekanntmachung des Hinweises auf die Patenterteilung: **03.12.2025**
(21) Anmeldenummer: 23764842.3
(22) Anmeldetag: 29.08.2023
(51) Int. Cl.: A61F 2/16

(54) **INTRAOKULARLINSE MIT EINER ZWEI ARME AUFWEISENDEN ERSTEN HAPTIK UND EINER ZWEITEN HAPTIK**
INTRAOCULAR LENS HAVING A FIRST HAPTIC, WITH TWO ARMS, AND A SECOND HAPTIC
LENTILLE INTRAOCULAIRE AYANT UN PREMIER DISPOSITIF HAPTIQUE, AVEC DEUX BRAS, ET UN SECOND DISPOSITIF HAPTIQUE

(30) Priorität: 09.09.2022 DE 102022122962
(43) Veröffentlichungstag der Anmeldung: 16.07.2025
(73) Patentinhaber: Carl Zeiss Meditec AG, 07745 Jena (DE)
(72) Erfinder: DAMM, Niklas, 73447 Oberkochen (DE); SCHREIBER, Benjamin, 73447 Oberkochen (DE)
(74) Vertreter: PATERIS Patentanwälte PartmbB
(86) Internationale Anmeldenummer: PCT/EP2023/073695
(87) Internationale Veröffentlichungsnummer: WO 2024/052173

(56) Entgegenhaltungen:
- DE-A1- 102017 221 476
- US-A1- 2006 041 308
- US-A1- 2009 171 458
- US-A1- 2020 323 626
- US-B1- 6 398 809

## Beschreibung

Die Erfindung betrifft eine Intraokularlinse, die eine erste Haptik, die einen ersten Arm und einen zweiten Arm aufweist, und eine zweite Haptik aufweist. Die zweite Haptik kann einen dritten Arm und einen vierten Arm aufweisen.

Bei einer Kataraktbehandlung eines Auges wird eine natürliche Linse eines Auges durch eine künstliche Intraokularlinse ersetzt. Die Intraokularlinse weist einen Optikkörper und mehrere Haptiken auf, mittels denen der Optikkörper in dem Kapselsack des Auges befestigt wird. Die Größe des Kapselsacks kann stark von Person zu Person zu variieren. Insbesondere kann der Kapselsack bei einem stark kurzsichtigen Auge größer sein als bei einem normalsichtigen Auge. Herkömmlicherweise werden die Intraokularlinsen mit einer einheitlichen Größe der Haptik hergestellt. Dies kann dazu führen, dass bei eher großen Kapselsäcken der Kapselsack einen nur niedrigen Druck auf die Haptik ausübt, wodurch der Optikkörper in dem Kapselsack verlagern kann oder um seine optische Achse verschwenken kann. Bei eher kleinen Kapselsäcken kann eine Verformung des Kapselsackes, wie beispielsweise eine Ovalisierung, erfolgen. Dies kann dazu führen, dass der Kapselsack Falten bildet, in die Zellen migrieren können, wodurch sich das Risiko für die Bildung eines Nachstars erhöht.

In US 2020/0323626 A1 ist eine in-situ einstellbare Intraokularlinse beschrieben. US 2006/0041308 A1 offenbart eine Intraokularlinse mit Haptiken und zusätzlichen Befestigungsstrukturen. DE 10 2017 221 476 A1 offenbart eine Anordnung zur Implantation in den Sulcus Ciliaris. US 6398809 B1 offenbart eine Intraokularlinse, die in erster Linie für die Refraktionskorrektur bei phaken Augen gedacht ist, bei denen die natürliche Linse des Auges intakt bleibt. US 2009/0171458 A1 offenbart eine Intraokularlinse mit Akkomodation.

Aufgabe der Erfindung ist es daher, eine Intraokularlinse mit einer Haptik zu schaffen, die bei verschieden großen Kapselsäcken eine gute Befestigung des Optikkörpers bewirkt und gleichzeitig nicht zu einer Verformung des Kapselsacks führt. Die Aufgabe wird gelöst durch die Merkmale des unabhängigen Anspruchs 1. Die weiteren Ausführungsformen werden in den abhängigen Ansprüchen definiert.

Die erfindungsgemäße Intraokularlinse weist einen Optikkörper, eine erste Haptik und eine zweite Haptik auf. Der Optikkörper weist eine optische Achse und eine auf die optische Achse bezogene Umfangsrichtung auf. Die erste Haptik weist einen ersten Arm auf, der ein erstes Längsende des ersten Arms, das an dem Optikkörper befestigt ist, ein zweites Längsende des ersten Arms und einen ersten Entspannungszustand aufweist, in dem der erste Arm frei von einer mechanischen Spannung ist. Zudem weist die erste Haptik einen zweiten Arm auf, der ein erstes Längsende des zweiten Arms, das an dem Optikkörper befestigt ist, ein zweites Längsende des zweiten Arms und einen zweiten Entspannungszustand aufweist, in dem der zweite Arm frei von einer mechanischen Spannung ist. Der erste Arm weist einen ersten Punkt auf, der in dem ersten Entspannungszustand einen ersten Abstand von der optischen Achse hat und der in dem ersten Entspannungszustand derjenige Punkt auf dem ersten Arm ist, der den längsten Abstand von der optischen Achse hat. Der zweite Arm weist einen zweiten Punkt auf, der in dem zweiten Entspannungszustand einen zweiten Abstand von der optischen Achse hat und der in dem zweiten Entspannungszustand derjenige Punkt auf dem zweiten Arm ist, der den längsten Abstand von der optischen Achse hat, wobei der erste Abstand länger als der zweite Abstand ist. Die zweite Haptik ist an dem Optikkörper befestigt und der ersten Haptik abgewandt angeordnet. Zudem ist der erste Arm in der Umfangsrichtung gekrümmt ausgeführt und der zweite Arm ist entgegen der Umfangsrichtung gekrümmt ausgeführt, wobei eine erste Tangente an einem ersten Tangentenpunkt an dem ersten Arm an dessen dem zweiten Arm zugewandten Seite mit einer zweiten Tangente an einem zweiten Tangentenpunkt an dem zweiten Arm an dessen dem ersten Arm zugewandten Seite einen ersten Tangentenschnittpunkt bildet. Der erste Tangentenschnittpunkt hat einen geringeren Abstand zur optischen Achse des Optikkörpers als der erste Tangentenunkt oder der zweite Tangentenunkt, wobei der erste Tangentenpunkt am ersten Arm der am nächsten zum Optikkörper gelegene Punkt abseits eines Schaftes des ersten Armes ist, mittels dessen der erste Arm an dem Optikkörper befestigt ist. Der zweite Tangentenpunkt ist am zweiten Arm der am nächsten zum Optikkörper gelegene Punkt abseits eines Schaftes des zweiten Armes, mittels dessen der zweite Arm an dem Optikkörper befestigt ist.

Ist der Kapselsack eher groß, kontaktiert nur der erste Arm den Kapselsack und befestigt somit den Kapselsack an dem Optikkörper. Ist der Kapselsack eher klein, kontaktiert auch der zweite Arm den Kapselsack und sowohl der erste Arm als auch der zweite Arm befestigen den Optikkörper an dem Kapselsack. Der erste Arm sorgt für eine gute Befestigung des Optikkörpers an dem Kapselsack, wenn der Kapselsack eher groß ist. Dadurch, dass bei den eher kleinen Kapselsäcken der erste Arm und der zweite Arm den Kapselsack kontaktieren, kann eine von der ersten Haptik auf den Kapselsack übertragene Rückstellkraft gleichmäßiger verteilt werden als bei einer herkömmlichen Haptik, die nur einen Arm aufweist. Dadurch findet eine geringere Ovalisierung des Kapselsackes als bei der herkömmlichen Haptik statt, wodurch eine Wahrscheinlichkeit für die Entstehung eines Nachstars sinkt. Zudem steht eine größere Kontaktfläche der ersten Haptik mit dem Kapselsack als bei der herkömmlichen Haptik zur Verfügung, wodurch der Optikkörper aufgrund von Reibung der ersten Haptik mit dem Kapselsack besonders fest an dem Kapselsack angebracht ist.

Es kann zudem vorkommen, dass der Kapselsack Zonen aufweist, die schwächer ausgebildet sind als andere Zonen des Kapselsacks. Dadurch, dass der erste Arm und der zweite Arm vorgesehen sind, können die schwächer ausgebildeten Zonen des Kapselsacks mit einer größeren Wahrscheinlichkeit überbrückt werden, als wenn die erste Haptik nur den einen Arm aufweist.

Des Weiteren besteht bei eher kleinen Kapselsäcken das Problem, dass die Haptik stark gebogen wird und dass daher große Kräfte von der Haptik auf den Optikkörper übertragen werden. Dies kann dazu führen, dass sich der Optikkörper wölbt, wodurch Abbildungsfehler entstehen können. Bei der erfindungsgemäßen Intraokularlinse wird bei dem eher kleinen Kapselsack die Kraft sowohl von dem ersten Arm als auch von dem zweiten Arm an zwei verschiedenen Punkten auf den Optikkörper übertragen. Dadurch, dass die Kraft von dem ersten und dem zweiten Arm an verschiedenen Punkten übertragen wird, verringert sich bei einem kleinen Kapselsack das Risiko, dass sich der Optikkörper wölbt. Beispielsweise hat der erste Arm eine Kraftkomponente, die in Richtung des zweiten Arms gerichtet ist, und der zweite Arm hat eine Kraftkomponente, die in Richtung des ersten Arms gerichtet ist. Beide Kraftkomponenten können sich zumindest zum Teil kompensieren und damit nicht zum Wölben des Optikkörpers beitragen.

Erfindungsgemäß ist in der ersten Haptik in der Umfangsrichtung zuerst der zweite Arm und dann der erste Arm angeordnet.

Die Summe aus der Rückstellkraft des ersten Arms und der Rückstellkraft des zweiten Arms liegt, wenn der Kapselsack einen Durchmesser in einem Bereich von 9 mm bis 11 mm hat, in einem Bereich von 0,005 bis 2 mN, bevorzugt in einem Bereich von 0,05 bis 0,5 mN und besonders bevorzugt in einem Bereich von 0,1 bis 0,3 mN. Um auf diese Werte zu kommen, kann, weil bei den eher kleinen Kapselsäcken sowohl der erste Arm als auch der zweite Arm eine Rückstellkraft auf den Kapselsack ausüben, beispielsweise der erste Arm eine niedrigere Biegesteifigkeit als bei der herkömmlichen Haptik haben. Weist die Intraokularlinse zwei der Haptiken mit insgesamt vier Armen auf, so liegt die Summe aus der Rückstellkraft aller vier Arme, wenn der Kapselsack einen Durchmesser in einem Bereich von 9 mm bis 11 mm hat, in einem Bereich von 0,01 bis 4 mN, bevorzugt in einem Bereich von 0,1 bis 1 mN und besonders bevorzugt in einem Bereich von 0,2 bis 0,6 mN.

Es ist bevorzugt, dass der zweite Arm eine niedrigere Biegesteifigkeit als der erste Arm hat. Dadurch wird vorteilhaft erreicht, dass die von der ersten Haptik erzeugte Rückstellkraft nur geringfügig ansteigt, wenn zusätzlich zu dem ersten Arm der zweite Arm den Kapselsack kontaktiert. Dadurch können Spannungspitzen in dem Kapselsack vorteilhaft vermieden werden. Der zweite Arm kann mit der niedrigeren Biegesteifigkeit als der erste Arm ausgeführt werden, indem der zweite Arm beispielsweise dünner als der erste Arm ausgeführt ist und/oder indem der Werkstoff des zweiten Arms verschieden von dem Werkstoff des ersten Arms ist. Alternativ ist denkbar, dass der zweite Arm eine höhere Biegesteifigkeit als der erste Arm hat. Dies kann beispielsweise relevant sein, wenn bei einem eher kleinen Kapselsack eine gewisse Rückstellkraft der ersten Haptik erreicht werden soll.

Das zweite Längsende des ersten Arms ist bevorzugt an dem Optikkörper befestigt. Dadurch hat der erste Arm die Form einer Schleife. Alternativ ist es bevorzugt, dass das zweite Längsende des ersten Arms frei liegt. Somit ist der erste Arm C-förmig oder J-förmig. Das zweite Längsende des zweiten Arms ist bevorzugt an dem Optikkörper befestigt. Dadurch hat der zweite Arm die Form einer Schleife. Alternativ ist es bevorzugt, dass das zweite Längsende des zweiten Arms frei liegt. Somit ist der zweite Arm C-förmig oder J-förmig.

Dadurch, dass der erste Arm in der Umfangsrichtung gekrümmt ausgeführt ist und der zweite Arm entgegen der Umfangsrichtung gekrümmt ausgeführt ist, wird ein größerer Widerstand gegen eine Rotation des Optikkörpers als bei der herkömmlichen Haptik erreicht. Dadurch kann der Optikkörper besonders fest gegen ein Verschwenken um die optische Achse in dem Kapselsack befestigt werden. Auch ein Verkippen des Optikkörpers gegenüber einer optischen Achse des Auges und/oder ein Verlagern des Optikkörpers in Richtung der optischen Achse des Auges ist weniger wahrscheinlich als bei der herkömmlichen Haptik.

Der erste Arm weist bevorzugt einen ersten Vorsprung der ersten Haptik auf, der in der Umfangsrichtung oder entgegen der Umfangsrichtung von dem verbliebenen ersten Arm vorsteht. Dadurch wird sich der erste Arm vorzugsweise in einem Bereich verbiegen, der abseits des ersten Vorsprungs der ersten Haptik liegt. Indem gewählt wird, in welchem Bereich des ersten Arms der erste Vorsprung der ersten Haptik vorgesehen wird, kann gesteuert werden, in welchem Bereich des ersten Arms der erste Arm sich vorzugsweise verbiegt. Es ist bevorzugt, dass der erste Vorsprung der ersten Haptik beabstandet von dem ersten Längsende des ersten Arms und beabstandet von dem zweiten Längsende des ersten Arms angeordnet ist. Dadurch wird sich der erste Arm vorzugsweise in dem Bereich des ersten Längsendes des ersten Arms und des zweiten Längsendes des ersten Arms verbiegen.

Es ist bevorzugt, dass der zweite Arm einen zweiten Vorsprung der ersten Haptik aufweist, der in der Umfangsrichtung oder entgegen der Umfangsrichtung von dem verbliebenen zweiten Arm vorsteht. Dadurch wird sich der zweite Arm vorzugsweise in einem Bereich verbiegen, der abseits des zweiten Vorsprungs der ersten Haptik liegt. Indem gewählt wird, in welchem Bereich des zweiten Arms der zweite Vorsprung der ersten Haptik vorgesehen wird, kann gesteuert werden, in welchem Bereich des zweiten Arms sich der zweite Arm vorzugsweise verbiegt. Der zweite Vorsprung der ersten Haptik bildet bevorzugt das zweite Längsende des zweiten Arms. Dadurch wird sich der zweite Arm vorzugsweise in dem Bereich des ersten Längsendes des zweiten Arms verbiegen.

Der erste Arm weist bevorzugt ein erstes Durchgangsloch der ersten Haptik auf. Insbesondere ist das erste Durchgangsloch der ersten Haptik in dem ersten Vorsprung der ersten Haptik angeordnet. Der zweite Arm weist bevorzugt ein zweites Durchgangsloch der ersten Haptik auf. Insbesondere ist das zweite Durchgangsloch der ersten Haptik in dem zweiten Vorsprung der ersten Haptik angeordnet. Ein Arzt, der eine Kataraktbehandlung durchführt, kann mit einem Instrument in den ersten Vorsprung der ersten Haptik oder in den zweiten Vorsprung der ersten Haptik eingreifen und somit die Intraokularlinse in dem Kapselsack um die optische Achse verschwenken. Dies ist insbesondere relevant, wenn der Optikkörper ein torischer Optikkörper ist.

Der erste Abstand liegt erfindungsgemäß in einem Bereich von 5,5 mm bis 7,0 mm. Der zweite Abstand liegt erfindungsgemäß in einem Bereich von 3,5 mm bis 6,5 mm, insbesondere in einem Bereich von 4,5 mm bis 5,5 mm.

Es ist bevorzugt, dass die zweite Haptik einen dritten Arm, der ein erstes Längsende des dritten Arms, das an dem Optikkörper befestigt ist, ein zweites Längsende des dritten Arms und einen dritten Entspannungszustand aufweist, in dem der dritte Arm frei von einer mechanischen Spannung ist, und einen vierten Arm aufweist, der ein erstes Längsende des vierten Arms, das an dem Optikkörper befestigt ist, ein zweites Längsende des vierten Arms und einen vierten Entspannungszustand aufweist, in dem der vierte Arm frei von einer mechanischen Spannung ist. Der dritte Arm weist einen dritten Punkt auf, der in dem dritten Entspannungszustand einen dritten Abstand von der optischen Achse hat und der in dem dritten Entspannungszustand derjenige Punkt auf dem dritten Arm ist, der den längsten Abstand von der optischen Achse hat. Der vierte Arm weist einen vierten Punkt auf, der in dem vierten Entspannungszustand einen vierten Abstand von der optischen Achse hat und der in dem vierten Entspannungszustand derjenige Punkt auf dem vierten Arm ist, der den längsten Abstand von der optischen Achse hat. Der dritte Abstand ist länger als der vierte Abstand. Zudem ist der dritte Arm in der Umfangsrichtung gekrümmt ausgeführt und der vierte Arm ist entgegen der Umfangsrichtung gekrümmt ausgeführt, wobei eine dritte Tangente an einem dritten Tangentenpunkt an dem dritten Arm an dessen dem vierten Arm zugewandten Seite mit einer vierten Tangente an einem vierten Tangentenpunkt an dem vierten Arm an dessen dem dritten Arm zugewandten Seite einen zweiten Tangentenschnittpunkt bildet. Der zweite Tangentenschnittpunkt hat einen geringeren Abstand zur optischen Achse des Optikkörpers als der dritte Tangentenpunkt oder der vierte Tangentenpunkt, wobei der dritte Tangentenpunkt am dritten Arm der am nächsten zum Optikkörper gelegene Punkt abseits eines Schaftes des dritten Armes ist, mittels dessen der dritte Arm an dem Optikkörper befestigt ist, und der vierte Tangentenpunkt am vierten Arm der am nächsten zum Optikkörper gelegene Punkt abseits eines Schaftes des vierten Armes ist, mittels dessen der vierte Arm an dem Optikkörper befestigt ist.

Es ist bevorzugt, dass in der zweiten Haptik in der Umfangsrichtung zuerst der vierte Arm und dann der dritte Arm angeordnet ist.

Der vierte Arm hat bevorzugt eine niedrigere Biegesteifigkeit als der dritte Arm. Alternativ ist es denkbar, dass der vierte Arm eine höhere Biegesteifigkeit als der dritte Arm hat.

Das zweite Längsende des dritten Arms ist bevorzugt an dem Optikkörper befestigt. Dadurch hat der dritte Arm die Form einer Schleife. Alternativ ist es bevorzugt, dass das zweite Längsende des dritten Arms frei liegt. Somit ist der dritte Arm C-förmig oder J-förmig. Das zweite Längsende des vierten Arms ist bevorzugt an dem Optikkörper befestigt. Dadurch hat der vierte Arm die Form einer Schleife. Alternativ ist es bevorzugt, dass das zweite Längsende des vierten Arms frei liegt. Somit ist der vierte Arm C-förmig oder J-förmig.

Es ist bevorzugt, dass der dritte Arm einen ersten Vorsprung der zweiten Haptik aufweist, der in der Umfangsrichtung oder entgegen der Umfangsrichtung von dem verbliebenen dritten Arm vorsteht. Der erste Vorsprung der zweiten Haptik ist bevorzugt beabstandet von dem ersten Längsende des dritten Arms und beabstandet von dem zweiten Längsende des dritten Arms angeordnet. Der vierte Arm weist bevorzugt einen zweiten Vorsprung der zweiten Haptik auf, der in der Umfangsrichtung oder entgegen der Umfangsrichtung von dem verbliebenen vierten Arm vorsteht. Es ist bevorzugt, dass der zweite Vorsprung der zweiten Haptik das zweite Längsende des vierten Arms bildet.

Der dritte Arm weist bevorzugt ein erstes Durchgangsloch der zweiten Haptik auf und/oder der vierte Arm weist bevorzugt ein zweites Durchgangsloch der zweiten Haptik auf. Insbesondere ist das erste Durchgangsloch der zweiten Haptik in dem ersten Vorsprung der zweiten Haptik angeordnet und/oder das zweite Durchgangsloch der zweiten Haptik ist in dem zweiten Vorsprung der zweiten Haptik angeordnet.

Der dritte Abstand liegt bevorzugt in einem Bereich von 5,5 mm bis 7,0 mm. Der zweite Abstand liegt bevorzugt in einem Bereich von 3,5 mm bis 6,5 mm, insbesondere in einem Bereich von 4,5 mm bis 5,5 mm.

Es ist bevorzugt, dass der erste Abstand gleich dem dritten Abstand ist und der zweite Abstand gleich dem vierten Abstand ist. Es ist insbesondere denkbar, dass die Intraokularlinse symmetrisch bezüglich der optischen Achse ausgebildet ist.

Im Folgenden wird anhand der beigefügten schematischen Zeichnungen die Erfindung näher erläutert. Es zeigen
Figur 1 eine Draufsicht auf eine erste Ausführungsform einer erfindungsgemäßen Intraokularlinse,
Figur 2 eine Draufsicht auf eine zweite Ausführungsform der Intraokularlinse,
Figur 3 eine Draufsicht auf eine dritte Ausführungsform der Intraokularlinse in unterschiedlichen Spannungs-Zuständen der Haptiken,
Figur 4 eine Draufsicht auf die dritte Ausführungsform der Intraokularlinse in unbelastetem Spannungs-Zustand,
Figur 5 eine Draufsicht auf eine vierte Ausführungsform der Intraokularlinse und
Figur 6 eine Draufsicht auf die dritte Ausführungsform der Intraokularlinse.

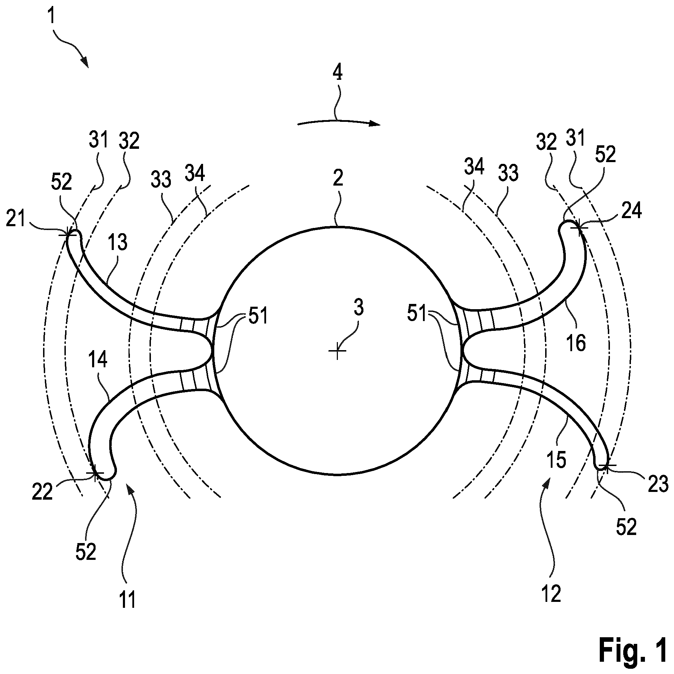

Wie es aus Figuren 1 bis 6 ersichtlich ist, weist eine Intraokularlinse 1 einen Optikkörper 2, eine erste Haptik 11 und eine zweite Haptik 12 auf. Der Optikkörper 2 weist eine optische Achse 3 und eine auf die optische Achse 3 bezogene Umfangsrichtung 4 auf. Die erste Haptik 11 weist einen ersten Arm 13 und einen zweiten Arm 14 auf. Der erste Arm 13 weist ein erstes Längsende 51 des ersten Arms 13, das an dem Optikkörper 2 befestigt ist, ein zweites Längsende 52 des ersten Arms 13 und einen ersten Entspannungszustand auf, in dem der erste Arm 13 frei von einer mechanischen Spannung ist. Der zweite Arm 14 weist ein erstes Längsende 51 des zweiten Arms 14, das an dem Optikkörper 2 befestigt ist, ein zweites Längsende 52 des zweiten Arms 14 und einen zweiten Entspannungszustand auf, in dem der zweite Arm 14 frei von einer mechanischen Spannung ist. Der erste Arm 13 weist einen ersten Punkt 21 auf, der in dem ersten Entspannungszustand einen ersten Abstand von der optischen Achse 3 hat und der in dem ersten Entspannungszustand derjenige Punkt auf dem ersten Arm 13 ist, der den längsten Abstand von der optischen Achse 3 hat. Der zweite Arm 14 weist einen zweiten Punkt 22 auf, der in dem zweiten Entspannungszustand einen zweiten Abstand von der optischen Achse 3 hat und der in dem zweiten Entspannungszustand derjenige Punkt auf dem zweiten Arm 14 ist, der den längsten Abstand von der optischen Achse 3 hat, wobei der erste Abstand länger als der zweite Abstand ist. Die zweite Haptik 12 ist an dem Optikkörper 2 befestigt ist und der ersten Haptik 11 abgewandt angeordnet. Der erste Arm 13 kann getrennt von dem zweiten Arm 14 angeordnet sein. Zudem ist denkbar, dass in dem ersten Entspannungszustand und dem zweiten Entspannungszustand mit zunehmendem Abstand von der optischen Achse 3 ein Abstand von dem ersten Arm 13 zu dem zweiten Arm 14 länger wird, insbesondere entlang des gesamten ersten Arms 13.

Figuren 1 bis 4 und 6 zeigen, dass die zweite Haptik 12 einen dritten Arm 15 und einen vierten Arm 16 aufweisen kann. Der dritte Arm 15 weist ein erstes Längsende 51 des dritten Arms 15, das an dem Optikkörper 2 befestigt ist, ein zweites Längsende 52 des dritten Arms 15 und einen dritten Entspannungszustand auf, in dem der dritte Arm 15 frei von einer mechanischen Spannung ist. Der vierte Arm 16 weist ein erstes Längsende 51 des vierten Arms 16, das an dem Optikkörper 2 befestigt ist, ein zweites Längsende 52 des vierten Arms 16 und einen vierten Entspannungszustand auf, in dem der vierte Arm 16 frei von einer mechanischen Spannung ist. Der dritte Arm 15 weist einen dritten Punkt 23 auf, der in dem dritten Entspannungszustand einen dritten Abstand von der optischen Achse 3 hat und der in dem dritten Entspannungszustand derjenige Punkt auf dem dritten Arm 15 ist, der den längsten Abstand von der optischen Achse 3 hat. Der vierte Arm 16 weist einen vierten Punkt 24 auf, der in dem vierten Entspannungszustand einen vierten Abstand von der optischen Achse 3 hat und der in dem vierten Entspannungszustand derjenige Punkt auf dem vierten Arm 16 ist, der den längsten Abstand von der optischen Achse 3 hat. Der dritte Abstand ist länger als der vierte Abstand. Der dritte Arm 15 kann getrennt von dem vierten Arm 16 angeordnet sein. Insbesondere können alle die Arme 13 bis 16 getrennt voneinander angeordnet sein und insbesondere vollständig getrennt voneinander angeordnet sein. Zudem ist denkbar, dass in dem dritten Entspannungszustand und dem vierten Entspannungszustand mit zunehmendem Abstand von der optischen Achse 3 ein Abstand von dem dritten Arm 15 zu dem vierten Arm 16 länger wird, insbesondere entlang des gesamten dritten Arms 15.

Figuren 1 bis 4 und 6 zeigen zudem, dass der erste Abstand gleich dem dritten Abstand sein kann und der zweite Abstand gleich dem vierten Abstand sein kann. Dies ist insbesondere daran zu erkennen, dass in Figuren 1 bis 3 ein erster Durchmesser 31 eingezeichnet ist, dessen Mittelpunkt auf der optischen Achse 3 liegt und auf dem der erste Punkt 21 und der dritte Punkt 23 liegen. Zudem ist ein zweiter Durchmesser 32 eingezeichnet, dessen Mittelpunkt auf der optischen Achse 3 liegt und auf dem der zweite Punkt 22 und der vierte Punkt 24 liegen.

Der erste Abstand und/oder der dritte Abstand, d.h. der Radius des Durchmessers 31, kann beispielsweise in einem Bereich von 5,5 mm bis 7,0 mm liegen. Der zweite Abstand und/oder der dritte Abstand, d.h. der Radius des Durchmessers 32, kann beispielweise in einem Bereich von 3,5 mm bis 6,5 mm liegen. Der Optikkörper 2 kann beispielsweise einen Durchmesser von 5 mm bis 7 mm haben, insbesondere von 5,5 mm bis 6,5 mm.

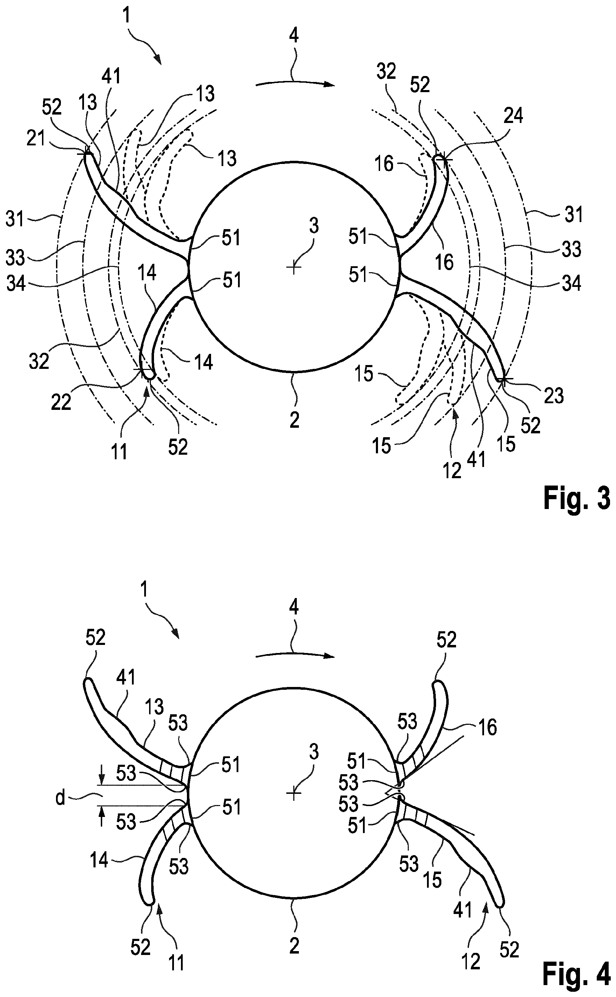

In Figur 3 ist die Intraokularlinse 1 in verschiedenen Zuständen dargestellt. Zum einen sind der erste Arm 13, der zweite Arm 14, der dritte Arm 15 und der vierte Arm 16 jeweils in ihren Entspannungszuständen dargestellt, in denen der erste Punkt 21 und der dritte Punkt 23 auf dem ersten Durchmesser 31 liegen und der zweite Punkt 22 und der vierte Punkt 24 auf dem zweiten Durchmesser 32 liegen. In Figur 3 ist ein erster Kapselsackdurchmesser 33 dargestellt, der zwischen dem ersten Durchmesser 31 und dem zweiten Durchmesser 32 liegt. Bei dem ersten Kapselsackdurchmesser 33 sind der erste Arm 13 aus dem ersten Entspannungszustand und der dritte Arm 15 aus dem dritten Entspannungszustand in Richtung zu dem Optikkörper 2 hin verlagert und der erste Arm 13 und der dritte Arm 15 üben aufgrund ihrer Biegesteifigkeit eine Rückstellkraft auf den Kapselsack aus. Der zweite Arm 14 befindet sich in dem zweiten Entspannungszustand und der vierte Arm 16 befindet sich in dem vierten Entspannungszustand. Ein zweiter Kapselsackdurchmesser 34 ist eingezeichnet, der kürzer als der zweite Durchmesser 32 ist. Dies führt dazu, dass der erste Arm 13 und der dritte Arm 15 weiter in Richtung zu dem Optikkörper 2 hin verlagert sind und zudem der zweite Arm 14 aus dem zweiten Entspannungszustand und der vierte Arm 16 aus dem vierten Entspannungszustand in Richtung zu dem Optikkörper 2 hin verlagert sind. Zusätzlich zu dem ersten Arm 13 und dem dritten Arm 15 üben nun der zweite Arm 14 und der vierte Arm 16 aufgrund ihrer Biegesteifigkeit eine Rückstellkraft auf den Kapselsack aus.

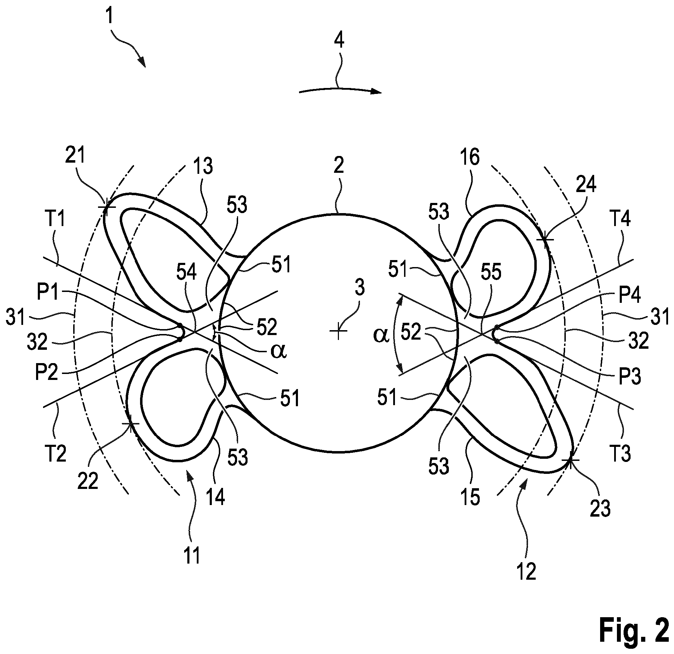

Bei den Ausführungsformen der Intraokularlinse 1 aus Figuren 1 und 3 bis 6 liegen das zweite Längsende 52 des ersten Arms 13, das zweite Längsende 52 des zweiten Arms 14, das zweite Längsende 52 des dritten Arms 15 und das zweite Längsende 52 des vierten Arms 16 frei. Der erste Arm 13, der zweite Arm 14, der dritte Arm 15 und der vierte Arm 16 können hierbei beispielsweise C-förmig oder J-förmig sein. Bei der Ausführungsform der Intraokularlinse 1 in Figur 2 sind das zweite Längsende 52 des ersten Arms 13, das zweite Längsende 52 des zweiten Arms 14, das zweite Längsende 52 des dritten Arms 15 und das zweite Längsende 52 des vierten Arms 16 an dem Optikkörper 2 befestigt. Dadurch bilden der erste Arm 13, der zweite Arm 14, der dritte Arm 15 und der vierte Arm 16 jeweils eine Schlaufe. Dabei ist denkbar, dass der erste Arm 13 eine höhere Biegesteifigkeit als der zweite Arm 14 hat und dass der der dritte Arm 15 eine höhere Biegesteifigkeit als der vierte Arm 15 hat.

Figuren 1 bis 6 zeigen, dass der erste Arm 13 und der dritte Arm 15 in der Umfangsrichtung 4 gekrümmt ausgeführt sein können und der zweite Arm 14 und der vierte Arm 16 entgegen der Umfangsrichtung 4 gekrümmt ausgeführt sein können. In der ersten Haptik 11 können in der Umfangsrichtung 4 zuerst der zweite Arm 14 und dann der erste Arm 13 angeordnet sein. In der zweiten Haptik 12 können in der Umfangsrichtung 4 zuerst der vierte Arm 16 und dann der dritte Arm 15 angeordnet sein. Bei den Ausführungsformen gemäß Figuren 1, 3, 4 und 6, bei denen das zweite Längsende 52 des jeweiligen Arms 13, 14, 15, 16 frei liegt, kann dies den vollständigen Arm betreffen. Bei der Ausführungsform gemäß Figur 2, bei denen das zweite Längsende 52 des jeweiligen Arms 13, 14, 15 und 15 an dem Optikkörper 2 befestigt ist, betrifft dies einen dem zweiten Arm 14 zugewandten Abschnitt des ersten Arms 13, einen dem ersten Arm 13 zugewandten Abschnitt des zweiten Arms 14, einen dem vierten Arm 16 zugewandten Abschnitt des dritten Arms 15 und einen dem dritten Arm 15 zugewandten Abschnitt des vierten Arms 16.

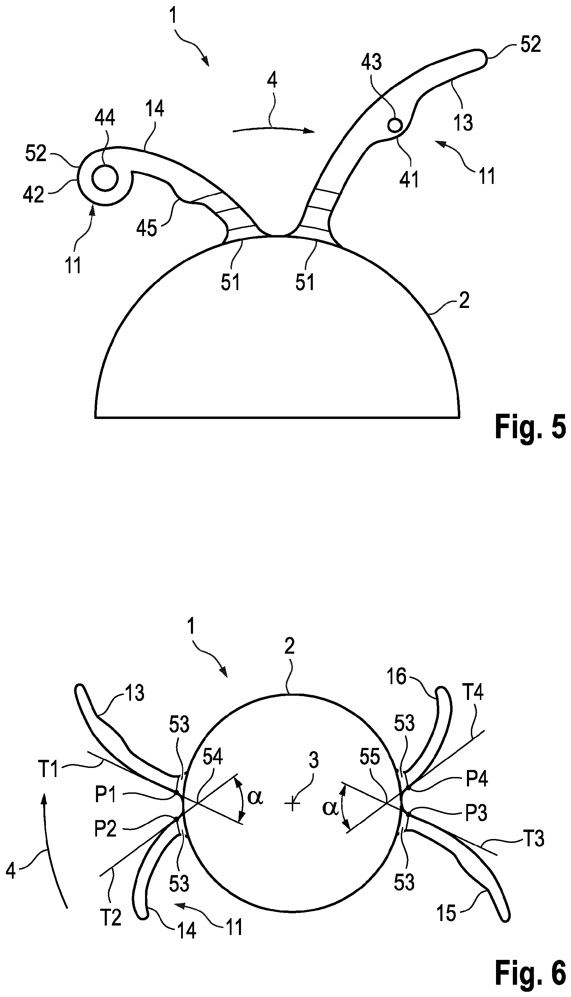

Wie es aus Figuren 3 bis 5 ersichtlich ist, kann der erste Arm 13 einen ersten Vorsprung 41 der ersten Haptik 11 aufweisen, der in der Umfangsrichtung 4 von dem verbliebenen ersten Arm 13 vorsteht. Der erste Vorsprung 41 der ersten Haptik 11 kann beabstandet von dem ersten Längsende 51 des ersten Arms 13 und beabstandet von dem zweiten Längsende 52 des ersten Arms 13 angeordnet sein. Der dritte Arm 15 kann einen ersten Vorsprung 41 der zweiten Haptik 12 aufweisen, der in der Umfangsrichtung 4 von dem verbliebenen dritten Arm 15 vorsteht. Der erste Vorsprung 41 der zweiten Haptik 12 kann beabstandet von dem ersten Längsende 51 des dritten Arms 15 und beabstandet von dem zweiten Längsende 52 des dritten Arms 15 angeordnet sein. Figur 5 zeigt, dass der zweite Arm 14 einen zweiten Vorsprung 42 der ersten Haptik 11 aufweisen kann, der entgegen der Umfangsrichtung 4 von dem verbliebenen zweiten Arm 14 vorsteht. Der zweite Vorsprung 42 der ersten Haptik 11 kann das zweite Längsende 52 des zweiten Arms 14 bilden. In analoger Weise kann der vierte Arm 16 einen zweiten Vorsprung 42 der zweiten Haptik 12 aufweisen, der entgegen der Umfangsrichtung 4 von dem verbliebenen vierten Arm 16 vorsteht. Der zweite Vorsprung 42 der zweiten Haptik 12 kann das zweite Längsende 52 des vierten Arms 16 bilden. Wie es aus Figur 5 ersichtlich ist, kann der zweite Arm 14 einen dritten Vorsprung 45 der ersten Haptik 11 aufweisen, der in der Umfangsrichtung 4 von dem verbliebenen zweiten Arm 14 vorsteht. Der dritte Vorsprung 45 der ersten Haptik 11 kann beabstandet von dem ersten Längsende 51 des zweiten Arms 14 und beabstandet von dem zweiten Längsende 52 des zweiten Arms 14 angeordnet sein. Der vierte Arm 16 kann einen dritten Vorsprung 45 der zweiten Haptik 12 aufweisen, der in der Umfangsrichtung 4 von dem verbliebenen vierten Arm 16 vorsteht. Der dritte Vorsprung 45 der zweiten Haptik 12 kann beabstandet von dem ersten Längsende 51 des vierten Arms 16 und beabstandet von dem zweiten Längsende 52 des vierten Arms 16 angeordnet sein. Es ist denkbar, dass der zweite Arm 14 nur den zweiten Vorsprung 42 der ersten Haptik 11, nur den dritten Vorsprung 45 der ersten Haptik 11 oder sowohl den zweiten Vorsprung 42 der ersten Haptik 11 als auch den dritten Vorsprung 45 der ersten Haptik 11 aufweist. In analoger Weise ist es denkbar, dass der vierte Arm 16 nur den zweiten Vorsprung 42 der zweiten Haptik 12, nur den dritten Vorsprung 45 der zweiten Haptik 12 oder sowohl den zweiten Vorsprung 42 der zweiten Haptik 12 als auch den dritten Vorsprung 45 der zweiten Haptik 11 aufweist.

Figur 5 zeigt, dass der erste Arm 13 ein erstes Durchgangsloch 43 der ersten Haptik 11 und der zweite Arm 14 ein zweites Durchgangsloch 44 der ersten Haptik 11 aufweisen kann. Das erste Durchgangsloch 43 der ersten Haptik 11 kann beispielsweise in dem ersten Vorsprung 41 der ersten Haptik 11 angeordnet sein und das zweite Durchgangsloch 44 der ersten Haptik 11 kann beispielsweise in dem zweiten Vorsprung 42 der ersten Haptik 11 angeordnet sein. In analoger Weise kann der dritte Arm 15 ein erstes Durchgangsloch 43 der zweiten Haptik 12 und der vierte Arm 16 ein zweites Durchgangsloch 44 der zweiten Haptik 12 aufweisen. Das erste Durchgangsloch 43 der zweiten Haptik 12 kann beispielsweise in dem ersten Vorsprung 41 der zweiten Haptik 12 angeordnet sein und das zweite Durchgangsloch 44 der zweiten Haptik 12 kann beispielsweise in dem zweiten Vorsprung 42 der zweiten Haptik 12 angeordnet sein. Das erste Durchgangsloch 43 der ersten Haptik 11, das zweite Durchgangsloch 44 der ersten Haptik 11, das erste Durchgangsloch 43 der zweiten Haptik 12 und/oder das zweite Durchgangsloch 44 der zweiten Haptik 12 können sich in Richtung der optischen Achse 3 vollständig durch den zugehörigen Arm 13 bis 16 erstrecken.

In Figuren 2 und 6 ist dargestellt, dass der erste Arm 13 und der zweite Arm 14 einen Winkel α einschließen. Der Winkel α kann konstruiert werden, indem an den ersten Arm 13 an dessen dem zweiten Arm 14 zugewandten Seite eine erste Tangente T1 an einem am nächsten zum Optikkörper 3 gelegenen ersten Tangentenpunkt P1 angelegt wird, der jedoch abseits eines Schaftes 53 des ersten Arms 13 liegt, mittels dessen der erste Arm 13 an dem Optikkörper 2 befestigt ist. Eine zweite Tangente T2 wird an den zweiten Arm 14 an dessen dem ersten Arm 13 zugewandten Seite an einem am nächsten zum Optikkörper 3 gelegenen zweiten Tangentenpunkt P2 angelegt, der jedoch abseits eines Schaftes 53 des zweiten Arms 14 liegt, mittels der der zweite Arm 14 an dem Optikkörper 2 befestigt ist. Die erste Tangente T1 und die zweite Tangente T2 schließen den Winkel α ein, wobei ein Tangentenschnittpunkt 54 der ersten Tangente T1 und der zweiten Tangente T2 einen geringeren Abstand zur optischen Achse 3 des Optikkörpers 2 als der erste Tangentenpunkt P1 oder der zweite Tangentenpunkt P2 hat. Bei Ausführungsformen, bei denen der erste Arm 13 und der zweite Arm 14 C-förmig oder J-förmig sind, kann der Winkel α beispielsweise in einem Bereich von 20° bis 140° liegen, insbesondere in einem Bereich von 20° bis 60° oder von 30° bis 50°.

Zudem ist in Figuren 2 und 6 dargestellt, dass der dritte Arm 15 und der vierte Arm 16 einen Winkel α einschließen. Der Winkel α kann konstruiert werden, indem an den dritten Arm 15 an dessen dem vierten Arm 16 zugewandten Seite eine dritte Tangente T3 an einem am nächsten zum Optikkörper 3 gelegenen dritten Tangentenpunkt P3 angelegt wird, der jedoch abseits eines Schaftes 53 des dritten Arms 15 liegt, mittels dessen der dritte Arm 15 an dem Optikkörper 2 befestigt ist. Eine vierte Tangente T4 wird an den vierten Arm 16 an dessen dem dritten Arm 15 zugewandten Seite an einem am nächsten zum Optikkörper 3 gelegenen vierten Tangentenpunkt P4 angelegt, der jedoch abseits eines Schaftes 53 des vierten Arms 16 liegt, mittels der der vierte Arm 16 an dem Optikkörper 2 befestigt ist. Die dritte Tangente T3 und die vierte Tangente T4 schließen den Winkel α ein, wobei ein zweiter Tangentenschnittpunkt 55 der dritten Tangente T3 und der vierten Tangente T4 einen geringeren Abstand zur optischen Achse 3 des Optikkörpers 2 als der dritte Tangentenpunkt P3 oder der vierte Tangentenpunkt P4 hat. Bei Ausführungsformen, bei denen der erste Arm 13 und der zweite Arm 14 C-förmig oder J-förmig sind, kann der Winkel α beispielsweise in einem Bereich von 20° bis 140° liegen, insbesondere in einem Bereich von 20° bis 60° oder von 30° bis 50°.

Figur 4 zeigt, dass der erste Arm 13 einen Abstand d von dem zweiten Arm 14 haben kann. Der Abstand d ist definiert als der kürzeste Abstand zwischen dem ersten Arm 13 und dem zweiten Arm 14 abseits eines Schaftes 53 des ersten Arms 13, mittels der der erste Arm 13 an dem Optikkörper 2 befestigt ist, und abseits eines Schaftes 53 des zweiten Arms 14, mittels der der zweite Arm 14 an dem Optikkörper 2 befestigt ist. Der Abstand d kann beispielsweise maximal 0,01 mm bis 6,0 mm, insbesondere maximal 0,1 mm bis 4,0 mm oder maximal 0,1 mm bis 1,0 mm betragen. In analoger Weise kann der dritte Arm 15 einen Abstand d von dem vierten Arm 16 haben. Der Abstand d ist definiert als der kürzeste Abstand zwischen dem dritten Arm 15 und dem vierten Arm 16 abseits eines Schaftes 53 des dritten Arms 15, mittels der der dritte Arm 15 an dem Optikkörper 2 befestigt ist, und abseits eines Schaftes 53 des vierten Arms 16, mittels der der vierte Arm 16 an dem Optikkörper 2 befestigt ist. Der Abstand d kann beispielsweise maximal 2 mm, insbesondere maximal 1 mm oder maximal 0,5 mm betragen.

### Bezugszeichenliste

1 Intraokularlinse
2 Optikkörper
3 optische Achse
4 Umfangsrichtung
11 erste Haptik
12 zweite Haptik
13 erster Arm
14 zweiter Arm
15 dritter Arm
16 vierter Arm
21 erster Punkt
22 zweiter Punkt
23 dritter Punkt
24 vierter Punkt
31 erster Durchmesser
32 zweiter Durchmesser
33 erster Kapselsackdurchmesser
34 zweiter Kapselsackdurchmesser
41 erster Vorsprung
42 zweiter Vorsprung
43 erstes Durchgangsloch
44 zweites Durchgangsloch
45 dritter Vorsprung
51 erstes Längsende
52 zweites Längsende
53 Schaft
54 Tangentenschnittpunkt
55 zweiter Tangentenschnittpunkt
α Winkel
d Abstand
P1 erster Tangentenpunkt an Tangente T1
P2 zweiter Tangentenpunkt an Tangente T2
P3 dritter Tangentenpunkt an dritter Tangente T3
P4 vierter Tangentenpunkt an vierter Tangente T4
T1 erste Tangente
T2 zweite Tangente
T3 dritte Tangente
T4 vierte Tangente

## Patentansprüche

1. Intraokularlinse mit einem Optikkörper (2), der eine optische Achse (3) und eine auf die optische Achse (3) bezogene Umfangsrichtung (4) aufweist, einer ersten Haptik (11), die einen ersten Arm (13), der ein erstes Längsende (51) des ersten Arms (13), das an dem Optikkörper (2) befestigt ist, ein zweites Längsende (52) des ersten Arms (13) und einen ersten Entspannungszustand aufweist, in dem der erste Arm (13) frei von einer mechanischen Spannung ist, und einen zweiten Arm (14) aufweist, der ein erstes Längsende (51) des zweiten Arms (14), das an dem Optikkörper (2) befestigt ist, ein zweites Längsende (52) des zweiten Arms (14) und einen zweiten Entspannungszustand aufweist, in dem der zweite Arm (14) frei von einer mechanischen Spannung ist, wobei der erste Arm (13) einen ersten Punkt (21) aufweist, der in dem ersten Entspannungszustand einen ersten Abstand von der optischen Achse (3) hat und der in dem ersten Entspannungszustand derjenige Punkt auf dem ersten Arm (13) ist, der den längsten Abstand von der optischen Achse (3) hat, wobei der zweite Arm (14) einen zweiten Punkt (22) aufweist, der in dem zweiten Entspannungszustand einen zweiten Abstand von der optischen Achse (3) hat und der in dem zweiten Entspannungszustand derjenige Punkt auf dem zweiten Arm (14) ist, der den längsten Abstand von der optischen Achse (3) hat, wobei der erste Abstand länger als der zweite Abstand ist, und einer zweiten Haptik (12), die an dem Optikkörper (2) befestigt ist und der ersten Haptik (11) abgewandt angeordnet ist, wobei der erste Arm (13) in der Umfangsrichtung (4) gekrümmt ausgeführt ist und der zweite Arm (14) entgegen der Umfangsrichtung (4) gekrümmt ausgeführt ist, wobei eine erste Tangente (T1) an einem ersten Tangentenpunkt (P1) an dem ersten Arm (13) an dessen dem zweiten Arm (14) zugewandten Seite mit einer zweiten Tangente (T2) an einem zweiten Tangentenpunkt (P2) an dem zweiten Arm (14) an dessen dem ersten Arm (13) zugewandten Seite einen ersten Tangentenschnittpunkt (54) bildet, wobei der erste Tangentenschnittpunkt (54) einen geringeren Abstand zur optischen Achse (3) des Optikkörpers (2) als der erste Tangentenpunkt (P1) oder der zweite Tangentenpunkt (P2) hat, wobei der erste Tangentenpunkt (P1) am ersten Arm (13) der am nächsten zum Optikkörper (2) gelegene Punkt abseits eines Schaftes (53) des ersten Armes (13), mittels dessen der erste Arm (13) an dem Optikkörper (2) befestigt ist, und der zweite Tangentenpunkt (P2) am zweiten Arm (14) der am nächsten zum Optikkörper (2) gelegene Punkt abseits eines Schaftes (53) des zweiten Armes (14), mittels dessen der zweite Arm (14) an dem Optikkörper (2) befestigt ist, ist, **dadurch gekennzeichnet, dass** der erste Arm (13) und der zweite Arm (14) eingerichtet sind, dass bei einem eher großem Kapselsack nur der erste Arm (13) den Kapselsack kontaktiert und bei einem eher kleinen Kapselsack sowohl der erste Arm (13) als auch der zweite Arm (14) den Kapselsack kontaktieren, wobei der erste Abstand in einem Bereich von 5,5 mm bis 7,0 mm liegt und der zweite Abstand in einem Bereich von 3,5 mm bis 6,5 mm liegt, wobei in der ersten Haptik (11) in der Umfangsrichtung (4) zuerst der zweite Arm (14) und dann der erste Arm (13) angeordnet ist.

2. Intraokularlinse gemäß Anspruch 1, wobei der zweite Arm (14) eine niedrigere Biegesteifigkeit als der erste Arm (13) hat.

3. Intraokularlinse gemäß Anspruch 1 oder 2, wobei das zweite Längsende (52) des ersten Arms (13) an dem Optikkörper (2) befestigt ist oder frei liegt.

4. Intraokularlinse gemäß einem der Ansprüche 1 bis 3, wobei das zweite Längsende (52) des zweiten Arms (14) an dem Optikkörper (2) befestigt ist oder frei liegt.

5. Intraokularlinse gemäß einem der Ansprüche 1 bis 4, wobei der erste Arm (13) und der zweite Arm (14) einen Winkel (α) einschließen, der in einem Bereich von 20° bis 140° liegt.

6. Intraokularlinse gemäß einem der Ansprüche 1 bis 5, wobei der erste Arm (13) einen Abstand (d) von dem zweiten Arm (14) hat, der maximal 2 mm beträgt und definiert ist als der kürzeste Abstand zwischen dem ersten Arm (13) und dem zweiten Arm (14) abseits eines Schaftes (53) des ersten Arms (13), mittels der der erste Arm (13) an dem Optikkörper (2) befestigt ist, und abseits eines Schaftes (53) des zweiten Arms (14), mittels der der zweite Arm (14) an dem Optikkörper (2) befestigt ist.

7. Intraokularlinse gemäß einem der Ansprüche 1 bis 6, wobei die zweite Haptik (12) einen dritten Arm (15), der ein erstes Längsende (51) des dritten Arms (15), das an dem Optikkörper (2) befestigt ist, ein zweites Längsende (52) des dritten Arms (15) und einen dritten Entspannungszustand aufweist, in dem der dritte Arm (15) frei von einer mechanischen Spannung ist, und einen vierten Arm (16) aufweist, der ein erstes Längsende (51) des vierten Arms (16), das an dem Optikkörper (2) befestigt ist, ein zweites Längsende (52) des vierten Arms (16) und einen vierten Entspannungszustand aufweist, in dem der vierte Arm (16) frei von einer mechanischen Spannung ist, wobei der dritte Arm (15) einen dritten Punkt (23) aufweist, der in dem dritten Entspannungszustand einen dritten Abstand von der optischen Achse (3) hat und der in dem dritten Entspannungszustand derjenige Punkt auf dem dritten Arm (15) ist, der den längsten Abstand von der optischen Achse (3) hat, wobei der vierte Arm (16) einen vierten Punkt (24) aufweist, der in dem vierten Entspannungszustand einen vierten Abstand von der optischen Achse (3) hat und der in dem vierten Entspannungszustand derjenige Punkt auf dem vierten Arm (16) ist, der den längsten Abstand von der optischen Achse (3) hat, wobei der dritte Abstand länger als der vierte Abstand ist, wobei der dritte Arm (15) in der Umfangsrichtung (4) gekrümmt ausgeführt ist und der vierte Arm (16) entgegen der Umfangsrichtung (4) gekrümmt ausgeführt ist, wobei eine dritte Tangente (T3) an einem dritten Tangentenpunkt (P3) an dem dritten Arm (15) an dessen dem vierten Arm (16) zugewandten Seite mit einer vierten Tangente (T4) an einem vierten Tangentenpunkt (P4) an dem vierten Arm (16) an dessen dem dritten Arm (15) zugewandten Seite einen zweiten Tangentenschnittpunkt (55) bildet, wobei der zweite Tangentenschnittpunkt (55) einen geringeren Abstand zur optischen Achse (3) des Optikkörpers (2) als der dritte Tangentepunkt (P3) oder der vierte Tangentenpunkt (P4) hat, wobei der dritte Tangentenpunkt (P3) am dritten Arm (15) der am nächsten zum Optikkörper (2) gelegene Punkt abseits eines Schaftes (53) des dritten Armes (15), mittels dessen der dritte Arm (15) an dem Optikkörper (2) befestigt ist, und der vierte Tangentenpunkt (P4) am vierten Arm (14) der am nächsten zum Optikkörper (2) gelegene Punkt abseits eines Schaftes (53) des vierten Armes (16), mittels dessen der vierte Arm (16) an dem Optikkörper 2 befestigt ist, ist.

8. Intraokularlinse gemäß Anspruch 7, wobei der vierte Arm (16) eine niedrigere Biegesteifigkeit als der dritte Arm (15) hat.

9. Intraokularlinse gemäß Anspruch 7 oder 8, wobei der dritte Arm (15) und der vierte Arm (16) einen Winkel (α) einschließen, der in einem Bereich von 20° bis 140° liegt.

10. Intraokularlinse gemäß einem der Ansprüche 7 bis 9, wobei der erste Abstand gleich dem dritten Abstand ist und der zweite Abstand gleich dem vierten Abstand ist.

## Claims

1. Intraocular lens having an optics body (2), which comprises an optical axis (3) and a circumferential direction (4) relative to the optical axis (3), a first haptic (11) comprising a first arm (13), which comprises a first longitudinal end (51) of the first arm (13) secured to the optics body (2), a second longitudinal end (52) of the first arm (13) and a first relaxation state in which the first arm (13) is free from any mechanical stress, and a second arm (14), which comprises a first longitudinal end (51) of the second arm (14) secured to the optics body (2), a second longitudinal end (52) of the second arm (14) and a second relaxation state in which the second arm (14) is free from any mechanical stress, wherein the first arm (13) comprises a first point (21), which is at a first distance from the optical axis (3) in the first relaxation state and which in the first relaxation state is that point on the first arm (13) which is at the furthest distance from the optical axis (3), wherein the second arm (14) comprises a second point (22), which is at a second distance from the optical axis (3) in the second relaxation state and which in the second relaxation state is that point on the second arm (14) which is at the furthest distance from the optical axis (3), wherein the first distance is greater than the second distance, and a second haptic (12) which is secured to the optics body (2) and arranged facing away from the first haptic (11), wherein the first arm (13) is curved in the circumferential direction (4) and the second arm (14) is curved against the circumferential direction (4), wherein a first tangent (T1) at a first tangent point (P1) on the first arm (13) on the latter's side facing the second arm (14) forms a first tangent intersection point (54) with a second tangent (T2) at a second tangent point (P2) on the second arm (14) on the latter's side facing the first arm (13), wherein the first tangent intersection point (54) is at a shorter distance from the optical axis (3) of the optics body (2) than both the first tangent point (P1) and the second tangent point (P2), wherein the first tangent point (P1) on the first arm (13) is the point closest to the optics body (2) away from a shaft (53) of the first arm (13) by means of which the first arm (13) is secured to the optics body (2), and the second tangent point (P2) on the second arm (14) is the point closest to the optics body (2) away from a shaft (53) of the second arm (14) by means of which the second arm (14) is secured to the optics body (2), **characterized in that** the first arm (13) and the second arm (14) are configured such that only the first arm (13) is in contact with the capsular bag in the case of a relatively large capsular bag, and both the first arm (13) and the second arm (14) are in contact with the capsular bag in the case of a relatively small capsular bag, with the first distance ranging from 5.5 mm to 7.0 mm and the second distance ranging from 3.5 mm to 6.5 mm, first the second arm (14) and then the first arm (13) being arranged in the first haptic (11) in the circumferential direction (4).

2. Intraocular lens according to Claim 1, wherein the second arm (14) has a lower flexural rigidity than the first arm (13).

3. Intraocular lens according to Claim 1 or 2, wherein the second longitudinal end (52) of the first arm (13) is secured to the optics body (2) or lies freely.

4. Intraocular lens according to any of Claim 1 to 3, wherein the second longitudinal end (52) of the second arm (14) is secured to the optics body (2) or lies freely.

5. Intraocular lens according to any of Claims 1 to 4, wherein the first arm (13) and the second arm (14) make an angle (α) ranging from 20° to 140°.

6. Intraocular lens according to any of Claims 1 to 5, wherein the first arm (13) is at a distance (d) from the second arm (14) that is no more than 2 mm and defined as the shortest distance between the first arm (13) and the second arm (14) away from a shaft (53) of the first arm (13), by means of which the first arm (13) is secured to the optics body (2), and away from a shaft (53) of the second arm (14), by means of which the second arm (14) is secured to the optics body (2).

7. Intraocular lens according to any of Claims 1 to 6, wherein the second haptic (12) comprises a third arm (15), which comprises a first longitudinal end (51) of the third arm (15) secured to the optics body (2), a second longitudinal end (52) of the third arm (15) and a third relaxation state in which the third arm (15) is free from any mechanical stress, and a fourth arm (16), which comprises a first longitudinal end (51) of the fourth arm (16) secured to the optics body (2), a second longitudinal end (52) of the fourth arm (16) and a fourth relaxation state in which the fourth arm (16) is free from any mechanical stress, wherein the third arm (15) comprises a third point (23), which is at a third distance from the optical axis (3) in the third relaxation state and which in the third relaxation state is that point on the third arm (15) which is at the furthest distance from the optical axis (3), wherein the fourth arm (16) comprises a fourth point (24), which is at a fourth distance from the optical axis (3) in the fourth relaxation state and which in the fourth relaxation state is that point on the fourth arm (16) which is at the furthest distance from the optical axis (3), wherein the third distance is greater than the fourth distance, wherein the third arm (15) is curved in the circumferential direction (4) and the fourth arm (16) is curved against the circumferential direction (4), wherein a third tangent (T3) at a third tangent point (P3) on the third arm (15) on the latter's side facing the fourth arm (16) forms a second tangent intersection point (55) with a fourth tangent (T4) at a fourth tangent point (P4) on the fourth arm (16) on the latter's side facing the third arm (15), wherein the second tangent intersection point (55) is at a shorter distance from the optical axis (3) of the optics body (2) than both the third tangent point (P3) and the fourth tangent point (P4), wherein the third tangent point (P3) on the third arm (15) is the point closest to the optics body (2) away from a shaft (53) of the third arm (15) by means of which the third arm (15) is secured to the optics body (2), and the fourth tangent point (P4) on the fourth arm (14) is the point closest to the optics body (2) away from a shaft (53) of the fourth arm (16) by means of which the fourth arm (16) is secured to the optics body (2),

8. Intraocular lens according to Claim 7, wherein the fourth arm (16) has a lower flexural rigidity than the third arm (15).

9. Intraocular lens according to Claim 7 or 8, wherein the third arm (15) and the fourth arm (16) make an angle (α) ranging from 20° to 140°.

10. Intraocular lens according to any of Claims 7 to 9, wherein the first distance is the same as the third distance, and the second distance is the same as the fourth distance.

## Revendications

1. Lentille intraoculaire comportant un corps optique (2) qui présente un axe optique (3) et une direction circonférentielle (4) par rapport à l'axe optique (3), une première haptique (11) qui présente un premier bras (13) qui présente une première extrémité longitudinale (51) du premier bras (13) qui est fixée au corps optique (2), une deuxième extrémité longitudinale (52) du premier bras (13) et un premier état de détente dans lequel le premier bras (13) est exempt d'une tension mécanique, et un deuxième bras (14) qui présente une première extrémité longitudinale (51) du deuxième bras (14) qui est fixée au corps optique (2), une deuxième extrémité longitudinale (52) du deuxième bras (14) et un deuxième état de détente dans lequel le deuxième bras (14) est exempt d'une tension mécanique, le premier bras (13) présentant un premier point (21) qui, dans le premier état de détente, a une première distance par rapport à l'axe optique (3) et qui, dans le premier état de détente, est le point sur le premier bras (13) qui a la plus longue distance par rapport à l'axe optique (3), le deuxième bras (14) présentant un deuxième point (22) qui, dans le deuxième état de détente, présente une deuxième distance par rapport à l'axe optique (3) et qui, dans le deuxième état de détente, est le point sur le deuxième bras (14) qui a la plus longue distance par rapport à l'axe optique (3), la première distance étant plus longue que la deuxième distance, et une deuxième haptique (12) qui est fixée au corps optique (2) et est agencé à l'opposé de la première haptique (11), le premier bras (13) étant conçu sous forme courbée dans la direction circonférentielle (4) et le deuxième bras (14) étant conçu sous forme courbée à l'encontre de la direction circonférentielle (4), une première tangente (T1) en un premier point de tangence (P1) sur le premier bras (13) sur son côté tourné vers le deuxième bras (14) formant avec une deuxième tangente (T2) en un deuxième point de tangence (P2) sur le deuxième bras (14) sur son côté tourné vers le premier bras (13) un premier point d'intersection de tangentes (54), le premier point d'intersection de tangentes (54) ayant une distance plus petite par rapport à l'axe optique (3) du corps optique (2) que le premier point de tangence (P1) ou le deuxième point de tangence (P2), le premier point de tangence (P1) sur le premier bras (13) étant le point le plus proche du corps optique (2), à part une tige (53) du premier bras (13) au moyen de laquelle le premier bras (13) est fixé au corps optique (2), et le deuxième point de tangence (P2) sur le deuxième bras (14) étant le point le plus proche du corps optique (2), à part une tige (53) du deuxième bras (14), au moyen de laquelle le deuxième bras (14) est fixé au corps optique (2), **caractérisée en ce que** le premier bras (13) et le deuxième bras (14) sont adaptés de telle sorte que, dans le cas d'un sac capsulaire plutôt grand, seul le premier bras (13) entre en contact avec le sac capsulaire et, dans le cas d'un sac capsulaire plutôt petit, le premier bras (13) et le deuxième bras (14) entrent tous deux en contact avec le sac capsulaire, la première distance se situant dans une plage de 5,5 mm à 7,0 mm et la deuxième distance se situant dans une plage de 3,5 mm à 6,5 mm, le deuxième bras (14) étant agencé en premier dans la première haptique (11) dans la direction circonférentielle (4), puis le premier bras (13).

2. Lentille intraoculaire selon la revendication 1, dans laquelle le deuxième bras (14) présente une rigidité à la flexion inférieure à celle du premier bras (13).

3. Lentille intraoculaire selon la revendication 1 ou 2, dans laquelle la deuxième extrémité longitudinale (52) du premier bras (13) est fixée au corps optique (2) ou est libre.

4. Lentille intraoculaire selon l'une quelconque des revendications 1 à 3, dans laquelle la deuxième extrémité longitudinale (52) du deuxième bras (14) est fixée au corps optique (2) ou est libre.

5. Lentille intraoculaire selon l'une quelconque des revendications 1 à 4, dans laquelle le premier bras (13) et le deuxième bras (14) forment un angle (α) dans une plage de 20° à 140°.

6. Lentille intraoculaire selon l'une quelconque des revendications 1 à 5, dans laquelle le premier bras (13) a une distance (d) par rapport au deuxième bras (14) qui est au maximum de 2 mm et qui est définie comme la distance la plus courte entre le premier bras (13) et le deuxième bras (14) à part une tige (53) du premier bras (13) au moyen de laquelle le premier bras (13) est fixé au corps optique (2), et à part une tige (53) du deuxième bras (14), au moyen de laquelle le deuxième bras (14) est fixé au corps optique (2).

7. Lentille intraoculaire selon l'une quelconque des revendications 1 à 6, dans laquelle la deuxième haptique (12) présente un troisième bras (15) qui présente une première extrémité longitudinale (51) du troisième bras (15) qui est fixée au corps optique (2), une deuxième extrémité longitudinale (52) du troisième bras (15) et un troisième état de détente dans lequel le troisième bras (15) est exempt d'une tension mécanique, et un quatrième bras (16) qui présente une première extrémité longitudinale (51) du quatrième bras (16) qui est fixée au corps optique (2), une deuxième extrémité longitudinale (52) du quatrième bras (16) et un quatrième état de détente dans lequel le quatrième bras (16) est exempt d'une tension mécanique, le troisième bras (15) présentant un troisième point (23) qui, dans le troisième état de détente, a une troisième distance par rapport à l'axe optique (3) et qui, dans le troisième état de détente, est le point sur le troisième bras (15) qui a la plus longue distance par rapport à l'axe optique (3), le quatrième bras (16) présentant un quatrième point (24) qui, dans le quatrième état de détente, a une quatrième distance par rapport à l'axe optique (3) et qui, dans le quatrième état de détente, est le point sur le quatrième bras (16) qui a la plus longue distance par rapport à l'axe optique (3), la troisième distance étant plus longue que la quatrième distance, le troisième bras (15) étant conçu sous forme courbée dans la direction circonférentielle (4) et le quatrième bras (16) étant conçu sous forme courbée à l'encontre de la direction circonférentielle (4), une troisième tangente (T3) en un troisième point de tangence (P3) sur le troisième bras (15) sur son côté tourné vers le quatrième bras (16) formant avec une quatrième tangente (T4) en un quatrième point de tangence (P4) sur le quatrième bras (16) sur son côté tourné vers le troisième bras (15) un deuxième point d'intersection de tangentes (55), le deuxième point d'intersection de tangentes (55) ayant une distance plus petite par rapport à l'axe optique (3) du corps optique (2) que le troisième point de tangence (P3) ou le quatrième point de tangence (P4), le troisième point de tangence (P3) sur le troisième bras (15) étant le point le plus proche du corps optique (2) à part une tige (53) du troisième bras (15) au moyen de laquelle le troisième bras (15) est fixé au corps optique (2), et le quatrième point de tangence (P4) sur le quatrième bras (14) étant le point le plus proche du corps optique (2) à part une tige (53) du quatrième bras (16), au moyen duquel le quatrième bras (16) est fixé au corps optique (2).

8. Lentille intraoculaire selon la revendication 7, dans laquelle le quatrième bras (16) présente une rigidité à la flexion inférieure à celle du troisième bras (15).

9. Lentille intraoculaire selon la revendication 7 ou 8, dans laquelle le troisième bras (15) et le quatrième bras (16) forment un angle (α) dans une plage de 20° à 140°.

10. Lentille intraoculaire selon l'une quelconque des revendications 7 à 9, dans laquelle la première distance est égale à la troisième distance et la deuxième distance est égale à la quatrième distance.
